# FASCICULE DE BREVET EUROPEEN

(11) **EP 0 541 753 B1**
(45) Date de publication et mention de la délivrance du brevet: **26.02.1997**
(21) Numéro de dépôt: 92910881.9
(22) Date de dépôt: 30.04.1992
(51) Int. Cl.: C12N 15/48, C12N 15/62, C12N 15/86, C12N 5/10, A61K 39/21

(54) **NOUVEAU VARIANT gp160 NON-CLIVABLE, SOLUBLE, DE FORME HYBRIDE**
HYBRIDE, LÖSLICHE UND NICHT GESPALTENE GP160-VARIANTE
NOVEL HYBRID, SOLUBLE AND UNCLEAVED gp160 VARIANT

(30) Priorité: 02.05.1991 FR 9105392
(43) Date de publication de la demande: 19.05.1993
(73) Titulaire: TRANSGENE S.A., 67082 Strasbourg Cédex (FR)
(72) Inventeur: KIENY, Marie-Paule, F-67000 Strasbourg (FR)
(74) Mandataire: Warcoin, Jacques
(86) Numéro de dépôt international: FR9200394
(87) Numéro de publication internationale: WO9219742

(56) Documents cités:
- EP-A- 0 245 136
- EP-A- 0 314 534
- EP-A- 0 370 458
- WO-A-90/12880
- WO-A-91/02544
- WO-A-91/05864
- WO-A-91/07664
- WO-A-91/15238

## Description

La présente invention concerne une méthode pour construire des moyens nécessaires à la production de nouvelles molécules capables d'induire une réponse immuno-protectrice à l'encontre d'un virus responsable du syndrome d'immunodéficience acquise (SIDA).

Chez un individu, cette maladie se développe à la suite d'une infection des lymphocytes T-helper par un rétrovirus HIV (human immunodeficiency virus). Jusqu'à présent on a répertorié ces rétrovirus en deux grand types: le type HIV-1 qui sévit essentiellement en Europe et en Amérique du Nord et le type HIV-2 caractéristique des infections africaines. A l'intérieur d'un même type, les rétrovirus HIV présentent entre eux un cerain degré de variabilité qui se caractérise par exemple par une différence de trophisme cellulaire ou par des protéines virales légèrement différentes les unes des autres. C'est pourquoi, lorsqu'on souhaite se référer à un rétrovirus HIV particulier, on emploie de préférence le terme de "souche virale".

D'une manière générale, les rétrovirus HIV ont la structure suivante : la molécule d'ARN génomique et diverses protéines associées sont encapsulés dans une capside de nature protéique (nucléocapside). L'ensemble est protégé par une membrane d'origine cellulaire ayant incorporé la protéine d'enveloppe d'origine virale.

Cette protéine d'enveloppe, sous des formes diverses, est à ce jour considéré comme un élément thérapeutique potentiel d'un vaccin contre le Sida.

Dans les conditions naturelles, la protéine d'enveloppe (env) est initialement synthétisée sous forme d'un précurseur comportant à son extrémité N-terminale une séquence signal qui initie le passage du précurseur dans le réticulum endoplasmique (voie de secretion). Ce peptide signal est ensuite éliminé par clivage protéolytique. Le produit de ce clivage est une protéine appelée gp160 qui est elle-même par la suite clivée en une petite sous-unité gp40 et une grande sous-unité gp120. L'extrémité N-terminale de la gp120 correspond à l'extrémité N-terminale de la gp160 tandis que l'extrémité C-terminale de la gp40 correspond à l'extrémité C-terminale de la gp160.

Chacune des sous-unités est sécrétée à l'extérieur de la cellule infectée. Toutefois, la gp40 reste ancrée dans la membrane cellulaire par son domaine transmembranaire. Sa partie C-terminale (domaine intra-cytoplasmique) reste en contact avec le cytoplasme tandis que sa partie N-terminale (domaine extra-cytoplasmique) se trouve à la surface de la cellule. La sous-unité gp120 est relarguée à l'extérieur de la cellule où elle interagit avec le domaine extracellulaire de la sous-unité gp40. Les deux sous-unités de la protéine d'enveloppe restent ainsi associées sous forme de complexe.

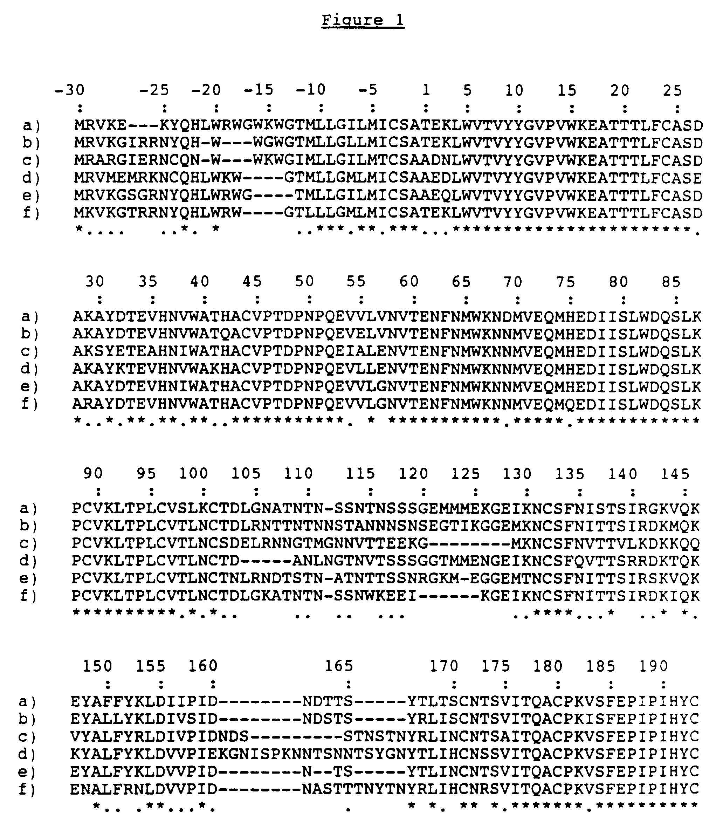

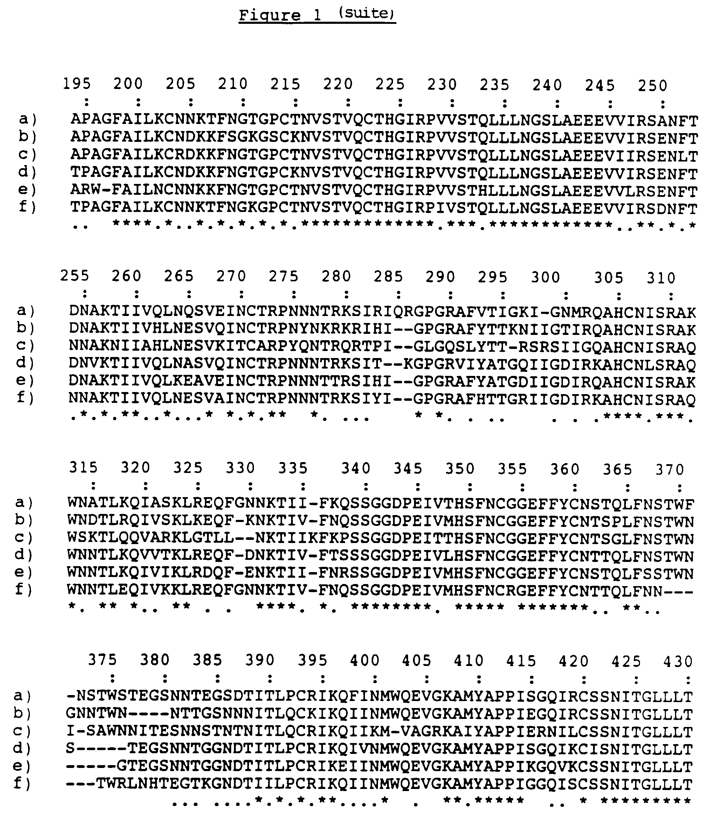

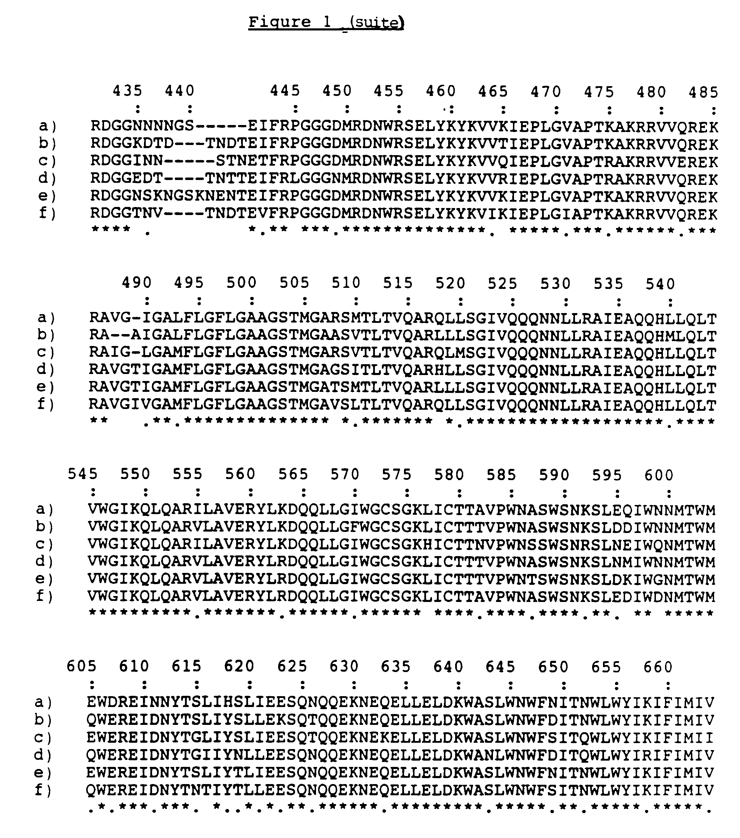

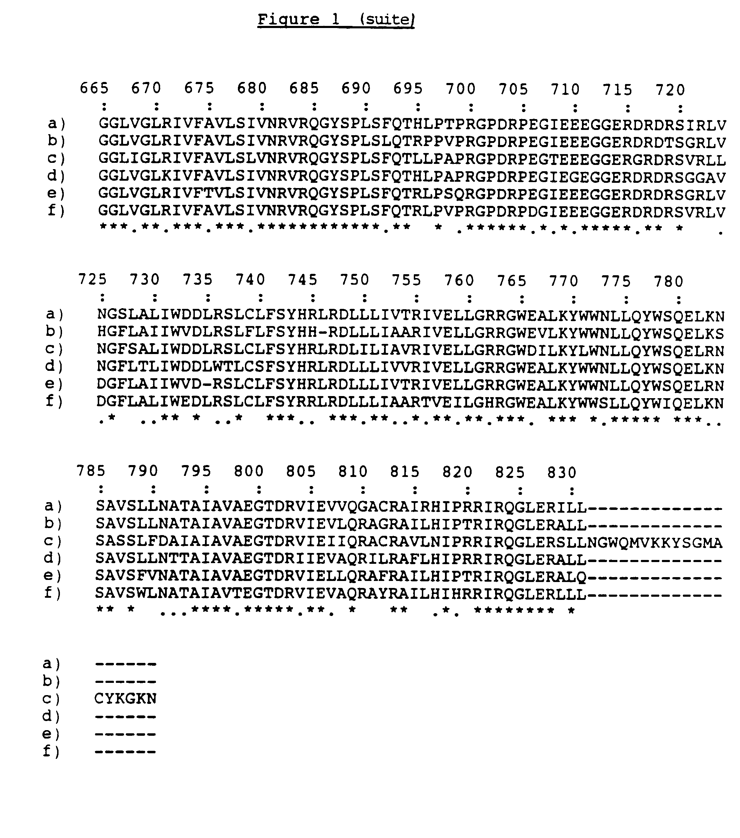

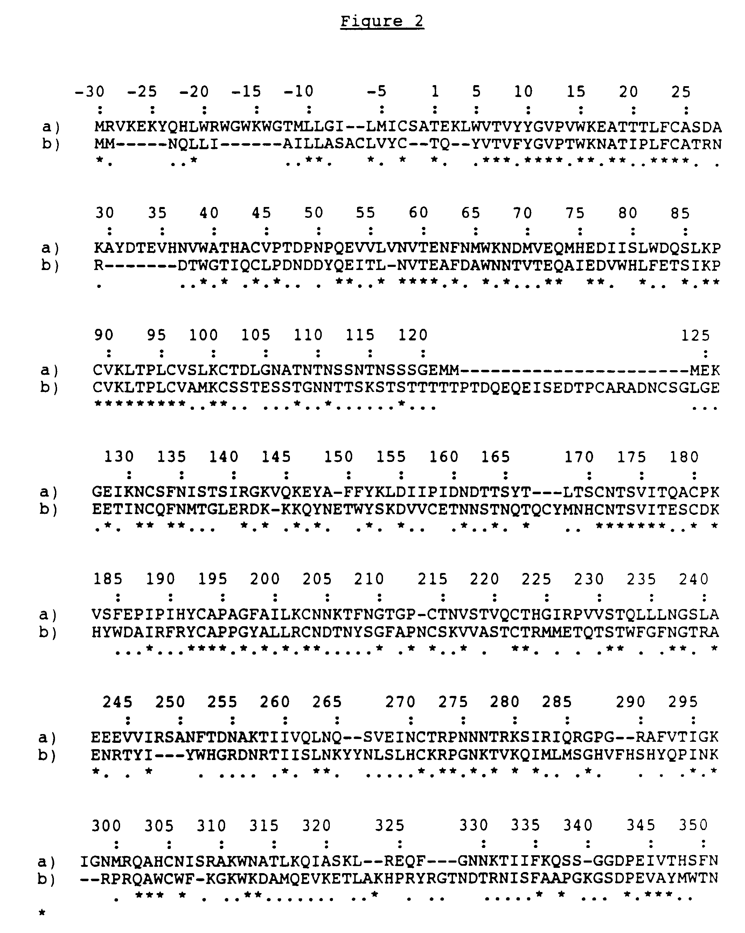

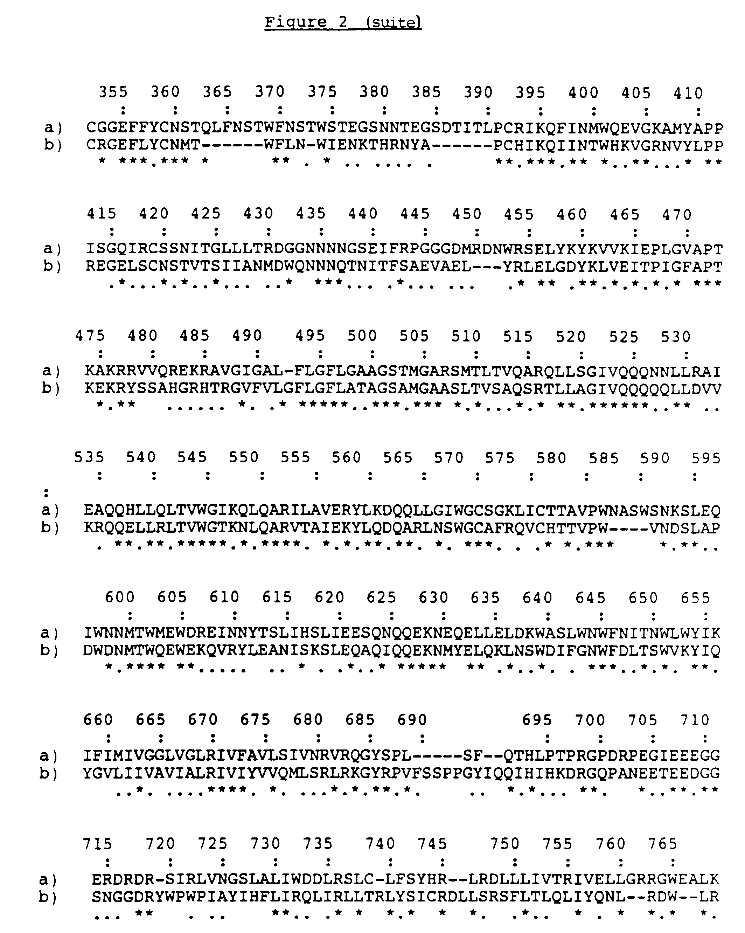

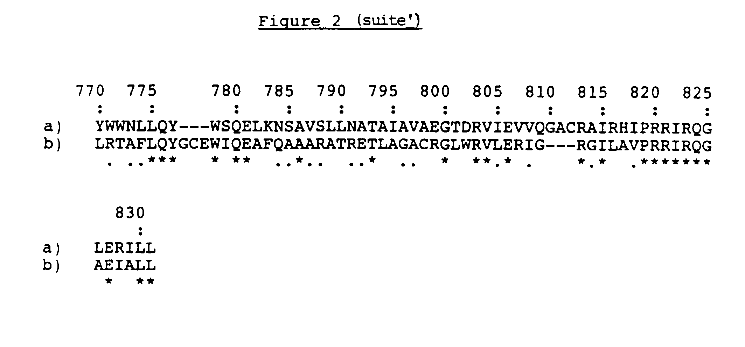

La séquence en acides aminés des précurseurs des protéines env de diverses souches virales sont maintenant connues. A titre d'exemple, la Figure 1 présente la séquence des précurseurs des gp160 des souches virales HIV-1 Bru, HIV-1 MN, HIV-1 ELI, HIV-1 RF, HIV-1 SF2C et HIV-1 SC. De même, la Figure 2 présente la séquence du précurseur de la gp160 de la souche virale HIV-2 Rod.

Par la suite et pour faciliter la compréhension, les séquences des gp160 autres que la gp160 de la souche HIV-1 Bru seront ci-après décrites par référence à la séquence de la gp160 de la souche HIV-1 Bru (ci-après appelée gp160-Bru) comme suit :
a) La gp160-Bru possède 831 acides aminés, ceux-ci numérotés de 1 à 831. En outre la gp120-Bru correspond à la séquence des 486 premiers acides aminés de la gp160-Bru tandis que la gp40-Bru correspond à la séquence commençant avec l'acide aminé en position 487 et finissant avec l'acide aminé en position 831.
b) La séquence d'une gp160 quelconque est alignée avec la séquence de la gp160-Bru de manière à présenter un maximum d'homologie. A cette fin, des emplacements vacants peuvent être introduits soit dans la séquence de la gp160-Bru, soit dans la séquence de la gp160 quelconque. Par définition, la position d'un acide aminé dans la gp160 quelconque sera spécifiée à l'identique par la position de l'acide aminé homologue dans la gp160-Bru.
c) En conséquence, lorsqu'un emplacement vacant est introduit dans la séquence de la gp160 quelconque, en face de l'acide aminé en position x, il n'existe pas d'acide aminé en position x dans la séquence de la gp160. Cependant, on considère que cet acide aminé jouit d'une présence virtuelle.
d) Lorsqu'un ou plusieurs emplacement(s) vacant(s) est (sont) introduit(s) dans la séquence de la gp160-Bru entre l'acide aminé en position x et l'acide aminé en position x + 1, on considère que, dans la gp160 quelconque, la position recouvre au moins deux sous-positions xₐ, x_{b}, ......, xₙ, chacune occupée par un acide aminé. Par définition, la position d'aval xₙ représente la position x.
e) Lorsqu'un ou plusieurs emplacement(s) vacant(s) est (sont) introduit(s) dans la séquence de la gp160-Bru en amont de l'acide aminé en position 1, on considère que, dans la gp160 quelconque, la position 1 recouvre au moins deux sous-positions 1ₐ, 1_{b}, ......, 1ₙ, chacune occupée par un acide aminé. Par définition, la position d'amont NH₂-terminale 1ₙ représente la position 1.
f) Lorsqu'un ou plusieurs emplacement(s) vacant(s) est (sont) introduit(s) dans la séquence de la gp160 quelconque en face du ou des acide(s) aminé(s) NH₂-terminal(aux) de la gp160-Bru, on indique que, par définition, l'acide aminé en position NH₂-terminale dans la gp160 quelconque cumule la position 1 et sa position d'acide aminé homologue.
g) Lorsqu'un ou plusieurs emplacement(s) vacant(s) est (sont) introduit(s) dans la séquence de la gp160 quelconque en face du ou des acide(s) aminé(s) COOH-terminal(aux) de la gp160-Bru, on indique que, par définition, l'acide aminé en position COOH-terminale dans la gp160 quelconque cumule la position 841 et sa position d'acide aminé homologue.

A ce jour, dans l'optique d'un vaccin, la protéine d'enveloppe est considérée comme un candidat potentiellement intéressant d'un point de vue thérapeutique. Toutefois, sa structure multichaîne d'origine constitue un handicap en terme de faisabilité. C'est pourquoi, il est apparu préférable d'utiliser une proteine simple chaîne qui conserverait la plupart des épitopes de la gp120 et ceux de la gp40. Ce type de protéine a déjà été proposé dans la demande de brevet WO 87/6260. Il s'agit plus particulièrement d'un variant gp160 non-clivable et soluble.

Toutes les gp160 des souches virales de type HIV-1 possèdent, en positions 483 - 486, un site de clivage dit majeur, reconnu par une ou des enzymes protéolytiques. Ce site de clivage est le même pour toutes les gp160 décrites à la Figure 1 et correspond à la séquence Arg-Glu-Lys-Arg (REKR). Les enzymes protéolytiques coupent immédiatement en aval de ce site de clivage pour libérer une gp120 et une gp40.

Lorsque ce site de clivage n'existe pas, on a démontré que les enzymes protéolytiques reconnaissent avec une efficacité toutefois moindre un site de clivage dit mineur et d'effectuer une coupure immédiatement en aval de celui-ci. Là aussi, ce site de clivage est le même pour toutes les gp160 montrées à la Figure 1. Il correspond à la séquence Lys-Arg-Arg (KRR) en positions 477 - 479, selon certains auteurs ou selon d'autres, à la séquence Lys-Ala-Lys-Arg (KAKR) en positions 475 - 478.

En outre, on indique que les gp160 des souches virales de type HIV-2 possèdent uniquement un site de clivage majeur en positions 475 - 478 qui correspond à la séquence Lys-Glu-Lys-Arg (KEKR).

Un variant gp160 non-clivable est un analogue artificiel d'une gp160 native. Sa séquence en acides aminés correspond à celle d'une gp160 native dans laquelle le site de clivage majeur et/ou le site de clivage mineur a (ont) été supprimé(s).

Une tel variant gp160 peut être synthétisé grâce aux techniques de l'ADN recombinant. Il suffit d'isoler un fragment d'ADN codant pour une gp160 native puis de modifier la région codant pour le site de clivage majeur par mutagenèse dirigée, de manière à obtenir un fragment d'ADN codant pour un variant gp160 insensible à une action protéolytique. Par la suite ce dernier fragment d'ADN est exprimé dans des conditions appropriées pour donner ledit variant gp160. Un tel variant gp160 qui ne contient plus le site de clivage majeur est appelée ci-après un variant gp160 non-clivable de type A.

De manière préférées la région codant pour le site de clivage mineur peut être en outre modifiée à des fins identiques. Dans ces conditions, on obtient un variant gp160 non-clivable de type A' qui ne contient ni le site de clivage majeur, ni le site de clivage mineur.

A cette fin, on indique à titre d'exemple que le site de clivage REKR, KEKR ou KAKR et le site de clivage KRR peuvent être respectivement remplacés par les séquences Asn-Glu-His-Gln (NEHQ) et Gln-Asn-His (QNH).

D'autre part, il est aussi nécessaire qu'une gp160 soit obtenue sous forme soluble. Une telle gp160 correspond à une gp160 native dans laquelle la région transmembranaire de nature hydrophobe a été supprimée. Cette région transmembranaire est située dans la zone correspondant à la gp40, du résidu acide aminé en position 659 au résidu acide aminé en position 680 De manière additionnelle mais superflue, une autre région hydrophobe, allant du résidu acide aminé en position 487 au résidu acide aminé en position 514 pourrait être délétée.

De façon similaire, les moyens nécessaires à la synthèse d'un variant gp160 soluble incluent bien évidemment le fragment d'ADN correspondant qui doit être obtenu par modification du fragment d'ADN original.

La comparaison des séquences des différentes gp160 déjà connues a mis en évidence au moins trois domaines dont la séquence est hypervariable d'une gp160 à l'autre. Ces trois domaines sont couramment appelés les domaines (ou boucles) V₁, V₂ et V₃.

Les deux premiers domaines V₁ et V₂ sont situés entre le résidu cystéine en position 96 et le résidu cystéine en position 171 tandis que le troisième domaine V₃ est situé du résidu cystéine en position 271 au résidu cystéine en position 306.

Il existe aussi un dernier domaine présentant un certain degré de variabilité, toutefois considéré comme moindre. Il s'agit du site de fixation au récepteur CD4 des lymphocytes T-helper; celui-ci étant situé approximativement du résidu acide aminé en position 340 au résidu acide aminé en position 440.

Quelque soit la gp160 considérée, des expériences de vaccination ont montré que le troisième domaine hypervariable est essentiel pour obtenir une immunité convenable. Toutefois, en raison de la nature hypervariable de ce domaine, la protection développée ne serait efficace qu'à l'encontre de la souche virale dont est issue la gp160 utilisée.

Enfin il semblerait que le premier et le deuxième domaines hypervariables ainsi que le site de fixation au récepteur CD4 influent sur le degré d'immunité que l'on pourrait obtenir.

Un vaccin d'un intérêt géneral devrait permettre de protéger les individus contre la majorité des souches virales HIV qui sévissent dans le monde. En conséquence, un vaccin à base de gp160 devrait contenir diverses gp160, chacune dérivée d'une souche virale différente. Afin de mettre en oeuvre un tel vaccin, il convient tout d'abord de construire les moyens destinés à la production du variant gp160 non-clivable et soluble, correspondant à chaque souche virale. En première approche, il s'agirait donc à chaque fois de cloner le fragment d'ADN codant pour la gp160 d'une souche virale déterminée, d'en déterminer sa séquence, de modifier ce fragment d'ADN afin de supprimer par substitution ou délétion des sites de clivage et des régions hydrophobes, puis enfin de placer ce fragment d'ADN ainsi modifié dans des conditions d'expression appropriées. Comme ce type d'opération devrait être renouvelée pour chaque gp160, la préparation d'un tel vaccin est prévue longue et coûteuse.

De plus, les souches virales peuvent varier au cours du temps e.g. par mutation. Telle souche, cause majeure de l'infection dans telle région du globe à tel moment, peut régresser en tant qu'agent infectieux et telle autre souche apparaître en remplacement. Il est donc important de pouvoir adapter rapidement un vaccin aux conditions épidémiologiques et, dans le cas présent, de disposer d'un variant gp160 dans les meilleurs délais.

A cette fin, on a maintenant trouvé une nouvelle méthode pour obtenir un variant gp160 approprié quelque soit la souche virale considérée. Dans la mesure où l'on dispose d'une cassette d'expression destinée à la production d'un variant gp160 non-clivable et soluble dérivé d'une première souche virale, cette méthode permet d'obtenir un variant similaire dérivé d'une deuxième souche virale en évitant le clonage complet du fragment d'ADN codant pour la gp160 de la deuxième souche virale et les modifications qui devraient s'en suivre.

En conséquence, l'invention propose une méthode pour construire une cassette d'expression comportant une unité d'ADN codant soit pour un précurseur d'un variant gp160 hybride non-clivable et soluble, soit pour un variant gp160 hybride non-clivable et soluble ; ledit variant ayant une séquence en acides aminés comprenant :
i) une première région dérivée de la gp160 d'une première souche du virus HIV et située dans cette dernière gp160 de l'acide aminé en position X à l'acide aminé en position Y, X étant un nombre de 1 à 271 et Y étant un nombre de 306 à 482 ;
ii) une deuxième région dérivée d'un variant gp160 soluble, non-clivable de type A ayant pour origine une deuxième souche du virus HIV et située dans ce dernier variant gp160 de l'acide aminé en position Y + 1 à l'acide aminé en position C-terminale, Y étant tel que défini ci-dessus ; et
iii) quand X est différent de 1, une troisième région dérivée de la gp160 de ladite deuxième souche du virus HIV et située dans cette dernière gp160 de l'acide aminé en position 1 à l'acide aminé en position X - 1, X étant tel que défini ci-dessus ;
ladite méthode comprenant :
a) l'acte de cloner un fragment d'ADN codant pour ladite première région ; et
b) l'acte d'insérer le fragment d'ADN cloné en a) dans un site d'une cassette qui comprend :
   m) en amont du site et en séquence :
      i) un promoteur,
      ii) un codon d'initiation de traduction,
      iii) en option, une première région d'ADN codant pour un peptide signal, et
      iv) quand X est tel que défini ci-dessus mais différent de 1, une deuxième région d'ADN codant pour ladite troisième région de la séquence en acides aminés dudit variant gp160 hybride ; et
   n) en aval du site et en séquence :
      i) quand Y est tel que défini ci-dessus, une troisième région d'ADN codant pour ladite deuxième région de la séquence en acides aminés dudit variant gp160 hybride et
      ii) un codon de terminaison de traduction ;
pour obtenir une cassette d'expression comportant ladite unité d'ADN.

Une telle cassette d'expression est l'outil indispensable qui permet de produire un variant gp160 hybride, soluble et non-clivable dans un système d'expression hétérologue.

Dans la méthode selon l'invention, le fragment d'ADN codant pour ladite première région peut être cloné selon n'importe quelle méthode en usage. On indique cependant que ce clonage peut être avantageusement effectué par la technique PCR à partir de l'ADN génomique de cellules infectées par ladite première souche du virus HIV. L'insertion du fragment d'ADN cloné s'effectue dans des sites de restriction initialement présents dans la cassette ou créés à cet effet, par exemple en utilisant un polylinker de manière appropriée. Une illustration de cette méthode est présentée par la suite dans les exemples.

Par "unité d'ADN codant pour un polypeptide quelconque", on entend un segment d'ADN dont le premier codon en position 5' et le dernier codon en position 3' codent respectivement pour le premier acide aminé en position N-terminale et le dernier acide aminé en position C-terminale du polypeptide.

Selon un mode préféré, la méthode selon l'invention est mise en oeuvre pour construire une cassette d'expression comportant une unité d'ADN codant pour un précurseur d'un variant gp160 hybride non-clivable et soluble.

Par "précurseur d'un variant gp160 selon l'invention", on signifie un polypeptide comportant un peptide signal et un variant gp160 mature ; l'extrémité N-terminale dudit variant étant associée à l'extrémité C-terminale du peptide signal par liaison peptidique. Ce précurseur est le produit initial de l'expression de l'unité d'ADN. Il est en particulier présent dans le cytoplasme de la cellule hôte, associé en position N-terminale avec un résidu methionine d'initiation de traduction. Le peptide signal permet d'initier le transfert du variant gp160 dans le réticulum endoplasmique. Lors de ce transfert, le peptide signal est abandonné par clivage protéolytique pour donner la forme mature du variant gp160. Dans la suite du texte, le terme "variant gp160" fait exclusivement référence à sa forme mature.

Le peptide signal peut être n'importe quel peptide signal en usage. Il doit être cependant choisi en tenant compte de l'organisme-hôte destiné à la production du variant gp160 selon l'invention. Par exemple, si l'organisme-hôte est une cellule de mammifère, le peptide signal peut être avantageusement sélectionné parmi les peptides signal des précurseurs des gp160 des différentes souches du virus HIV, indépendamment de l'origine des première, deuxième et troisième régions du variant selon l'invention. De manière alternative, on peut utiliser des peptides signal synthétiques. De tels peptides signal sont par exemple, des peptides signal hybrides dont l'extrémité N-terminale dérive du précurseur de la gp160 de ladite deuxième souche du virus HIV et dont l'extrémité C-terminale dérive du précurseur de la gp160 de ladite première souche du virus HIV. A titre indicatif, on mentionne de plus, que le peptide signal du précurseur de la glycoprotéine d'une souche du virus de la rage peut être aussi utilisé. Enfin. l'homme du métier doit comprendre que cette liste est non-limitative.

L'invention propose de même un variant gp160 hybride, non-clivable et soluble, qui comprend :
i) une première région dérivée de la gp160 d'une première souche du virus HIV et située dans cette dernière gp160 de l'acide aminé en position X à l'acide aminé en position Y, X étant un nombre de 1 à 271 et Y étant un nombre de 306 à 482 ;
ii) une deuxième région dérivée d'un variant gp160 soluble, non-clivable de type A ayant pour origine une deuxième souche du virus HIV et située dans ce dernier variant gp160 de l'acide aminé en position Y + 1 à l'acide aminé en position C-terminale, Y étant tel que défini ci-dessus ; et
iii) quand X est différent de 1, une troisième région dérivée de la gp160 de ladite deuxième souche du virus HIV et située dans cette dernière gp160 de l'acide aminé en position 1 à l'acide aminé en position X - 1, X étant tel que défini ci-dessus,

La première, ainsi que la deuxième et troisième régions du variant gp160 selon l'invention peuvent être dérivées de la gp160 de n'importe quelle souche du virus HIV, à condition que la première souche soit différente de la deuxième. De manière préférée, la première région dérive de la gp160 d'une souche virale sélectionnée parmi les souches HIV-2 Rod, HIV-1 Eli, HIV-1 RF, HIV-1 SF2C, HIV-1 SC et HIV-1 MN, cette dernière étant plus particulièrement préférée. De même, la deuxième et troisième régions ont de préférence pour origine la gp160 de la souche HIV-1 Bru.

Par "variant gp160 non-clivable de type A", on entend un variant gp160 :
- soit dérivé d'une gp160 d'une souche virale de type HIV-1 et ne contenant plus le site de clivage majeur,
- soit dérivé d'une gp160 d'une souche virale de type HIV-2 et ne contenant plus le site de clivage.
Lorsque le variant gp160 dérive d'une gp160 d'une souche virale de type HIV-1, ce variant est de préférence de type A' ; c'est-à-dire ne contenant ni le site de clivage majeur, ni le site de clivage mineur.

Par "variant gp160 soluble", on entend un variant gp160 dérivé d'une gp160 native qui ne contient plus de région transmembranaire ou dont la région transmembranaire native a été mutée de manière à ne plus pouvoir assurer sa fonction d'ancrage dans la membrane. De plus, un tel variant soluble peut avantageusement ne plus contenir de région hydrophobe.

Par "cassette d'expression", on entend un segment d'ADN comprenant une unité d'ADN à exprimer ainsi que les éléments nécessaires à l'expression de cette dernière. L'expression d'une unité d'ADN est obtenue par transcription de cette unité d'ADN en ARN messager et par traduction de cet ARN messager en protéine. Par conséquent, les éléments indispensables sont un promoteur constitutif ou inductible (initiation de la transcription), un codon d'initiation de traduction (codon ATG) et un codon de fin de traduction (codon TAG, TAA ou TGA). En outre, une cassette d'expression peut aussi contenir d'autres éléments ; par exemple, un terminateur de transcription.

L'homme du métier sait bien évidemment choisir le promoteur et le terminateur appropriés en fonction de l'organisme-hôte dans lequel on souhaite exprimer l'unité d'ADN et en fonction du vecteur dans lequel la cassette d'expression doit être insérée pour assurer sa réplication.

En accord avec ce qui précède, l'invention propose de même, une cassette d'expression comportant une unité d'ADN codant soit pour un précurseur d'un variant gp160 selon l'invention, soit pour un variant gp160 selon l'invention ainsi que les éléments nécessaires à expression de ladite unité d'ADN.

Afin d'assurer sa réplication autonome dans un organisme hôte, une cassette d'expression selon l'invention peut être insérée dans différents types de vecteur ayant une origine de réplication adaptée à l'organisme hôte ; par exemple un plasmide ou un virus. Les vecteurs de type viral ont en particulier la capacité d'intégrer dans leur génome une quantité substantielle d'ADN étranger sans nuire à leur capacité de réplication. Parmi ceux-ci, on compte par exemple, les poxvirus, tels que le virus de la vaccine, le poxvirus du canari et la poxvirus de la variole aviaire ; les baculovirus tels que et les adénovirus tel que l'Adénovirus-2 ou l'Adénovirus-5.

Outre leur utilisation dans un système de production hétérologue, certains de ces vecteurs de type viral peuvent être fonctionnels à titre d'agent de vaccination. Il s'agit en particulier des poxvirus et des adénovirus.

C'est pourquoi, l'invention concerne de même un vecteur viral dans le génome duquel est inséré une cassette d'expression selon l'invention.

Sous un autre aspect de l'invention, on procure aussi une cellule transformée par une cassette d'expression selon l'invention. La cassette d'expression qui transforme la cellule peut être véhiculée par un plasmide ou, de manière alternative, être intégrée dans le génome de la cellule hôte.

L'organisme-hôte destiné à la production du variant gp160 selon l'invention peut être n'importe quel type de cellule, de préférence eucaryote. Ceci inclut par exemple : des champignons tels que les levures, des cellules d'insectes et des cellules de mammifères.

L'invention propose, en outre, deux procédés alternatifs visant la production d'un variant gp160 selon l'invention :
- le premier procédé comprend l'acte de cultiver une cellule selon l'invention et l'acte de récolter ledit variant à partir de la culture,
- le second procédé comprend l'acte d'infecter une culture de cellules avec un vecteur viral selon l'invention et l'acte de récolter ledit variant à partir de la culture.

Un variant gp160 ainsi qu'un vecteur viral selon l'invention possèdent une activité vaccinale à l'encontre d'un virus HIV et, par conséquent, sont utiles à titre de produits pharmaceutiques en particulier destinés au traitement ou à la prévention du Sida.

En conséquence, l'invention propose :
i) une composition pharmaceutique destinée au traitement curatif ou préventif du Sida qui comprend à titre d'agent thérapeutique au moins un variant gp160 ou un vecteur viral selon l'invention,
ii) une méthode de traitement curatif ou préventif du Sida qui comprend l'acte d'administrer une quantité thérapeutiquement effective d'un variant gp160 ou d'un vecteur viral selon l'invention à un patient ayant besoin d'un tel traitement.
iii) l'usage d'un variant gp160 ou d'un vecteur viral selon l'invention à titre d'agent thérapeutique destiné au traitement curatif ou préventif du Sida.

De manière préférée, une composition pharmaceutique selon l'invention peut contenir plusieurs variants gp160 selon l'invention ; chaque variant gp160 possédant au moins un troisième domaine hypervariable (boucle V₃) différent des autres variants présents dans la composition. Une telle composition pharmaceutique devrait donc permettre de protéger correctement un individu vis-à-vis de diverses souches HIV.

Une composition pharmaceutique selon l'invention peut contenir en outre d'autres agents thérapeutiques comme, par exemple, un peptide correspondant essentiellement au troisième domaine hypervariable d'une gp160 (ci-après appelé peptide V₃). De préférence, un tel peptide a une séquence substantiellement identique à la séquence du troisième domaine d'un variant gp160 contenu dans la composition pharmaceutique selon l'invention. De manière similaire, plusieurs peptides V₃ peuvent être présents dans la composition. En particulier, si la composition pharmaceutique selon l'invention contient différents variants gp160, on peut bien évidemment y ajouter les peptides V₃ correspondants.

Une composition pharmaceutique selon l'invention peut être fabriquée de manière conventionnelle. En particulier, on associe un variant gp160 selon l'invention avec un diluant ou un support acceptable d'un point de vue pharmaceutique. Enfin, une composition selon l'invention peut contenir un adjuvant de vaccination tel que l'alun. De manière alternative, cet adjuvant peut être ajouté à une composition selon l'invention juste avant usage.

Une composition selon l'invention peut être administrée par n'importe quelle voie conventionnelle en usage dans le domaine des vaccins, en particulier par voie sous-cutanée, par exemple sous forme de solution ou de suspension injectable. L'administration peut avoir lieu en dose unique ou répétée une ou plusieurs fois après un certain délai d'intervalle. Le dosage approprié varie en fonction de divers paramètres, par exemple, de l'individu traité ou du mode d'administration.

De manière alternative, une composition pharmaceutique selon l'invention peut être présentée comme partie d'un kit de traitement. A titre d'exemple, on indique qu'un tel kit peut contenir :
- d'une part, une composition pharmaceutique contenant au moins un variant gp160 selon l'invention et,
- d'autre part, une composition pharmaceutique contenant au moins un peptide V₃,
- ainsi qu'une notice spécifiant les instructions relatives à l'administration séquentielle ou concomitante des compositions pharmaceutiques contenues dans le kit.

L'invention est illustrée ci-après par référence aux figures suivantes:

La Figure 1 présente la séquence des acides aminés des précurseurs des gp160 natives des souches virales HIV-1 Bru (a), HIV-1 MN (b), HIV-1 Eli (c), HIV-1 RF (d), HIV-1 SC (e) et HIV-1 SF2C (f). Les résidus acides aminés des séquences signal sont numérotés de la position -1 à -29. Le résidu méthionine en position -30 correspond au codon d'initiation de traduction. Les résidus acides aminés des gp160 matures sont numérotés de la position 1 à 841. L'astérisque (∗) symbolise l'identité des résidus acides aminés à une position donnée tandis que le point (.) indique un changement conservatif (acides aminés différents, mais apartenant à une même classe).

La Figure 2 présente la séquence des acides aminés des précurseurs des gp160 natives des souches virales HIV-1 Bru (a) et HIV-2 Rod (b). Les résidus acides aminés des séquences signal sont numérotés de la position -1 à -29. Le résidu méthionine en position -30 correspond au codon d'initiation de traduction. Les résidus acides aminés des gp160 matures sont numérotés de la position 1 à 841. L'astérisque (∗) symbolise l'identité des résidus acides aminés à une position donnée tandis que le point (.) indique un changement conservatif (acides aminés différents, mais apartenant à une même classe).

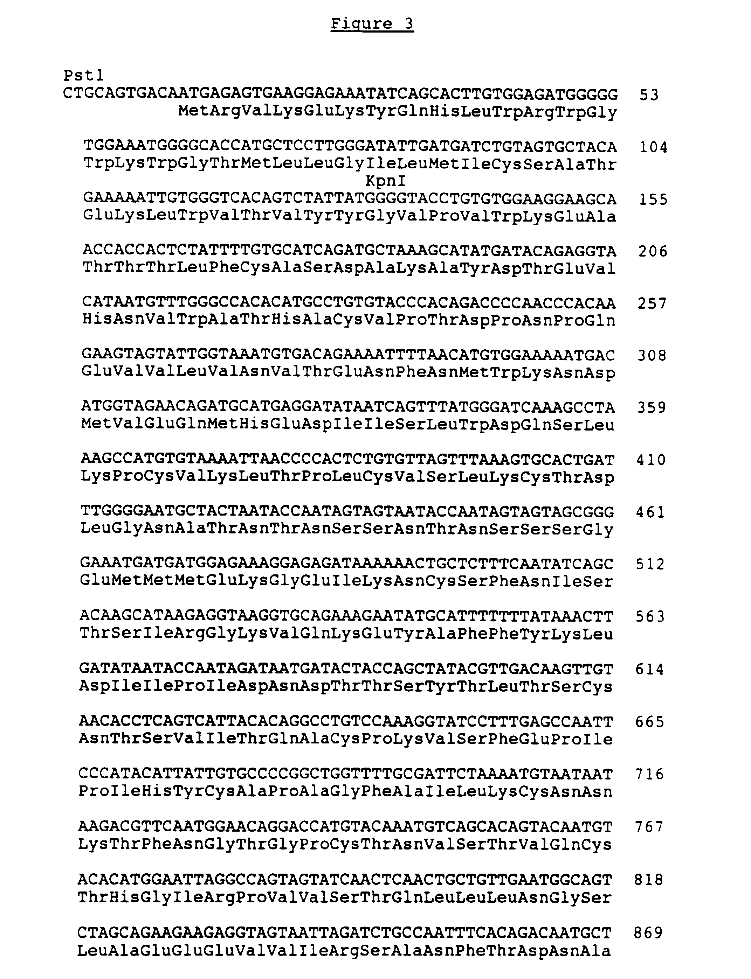

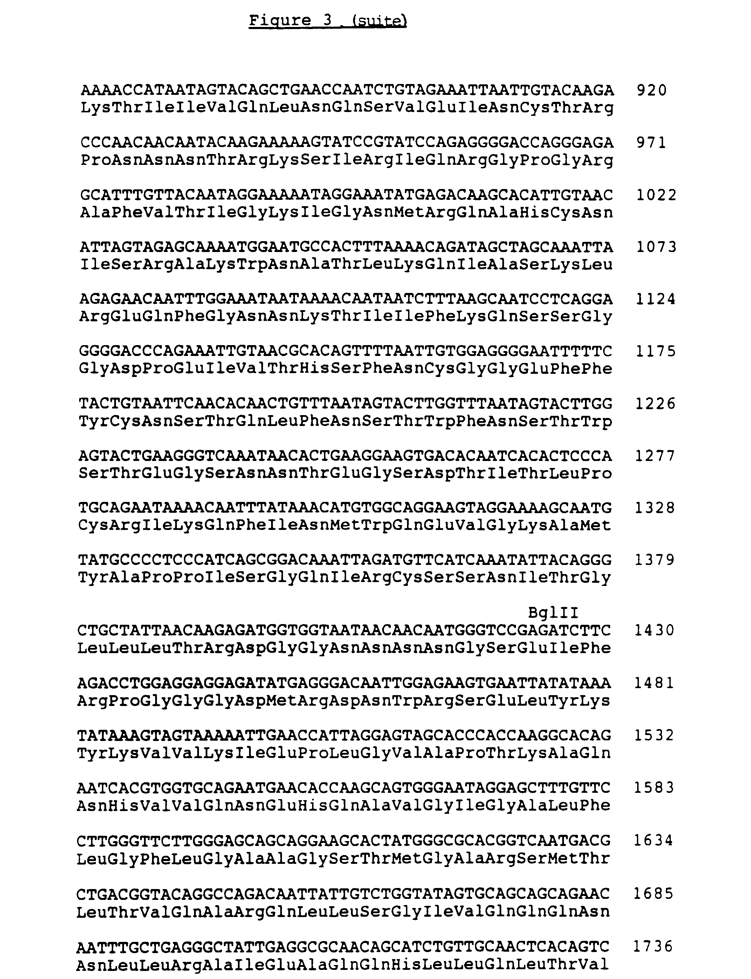

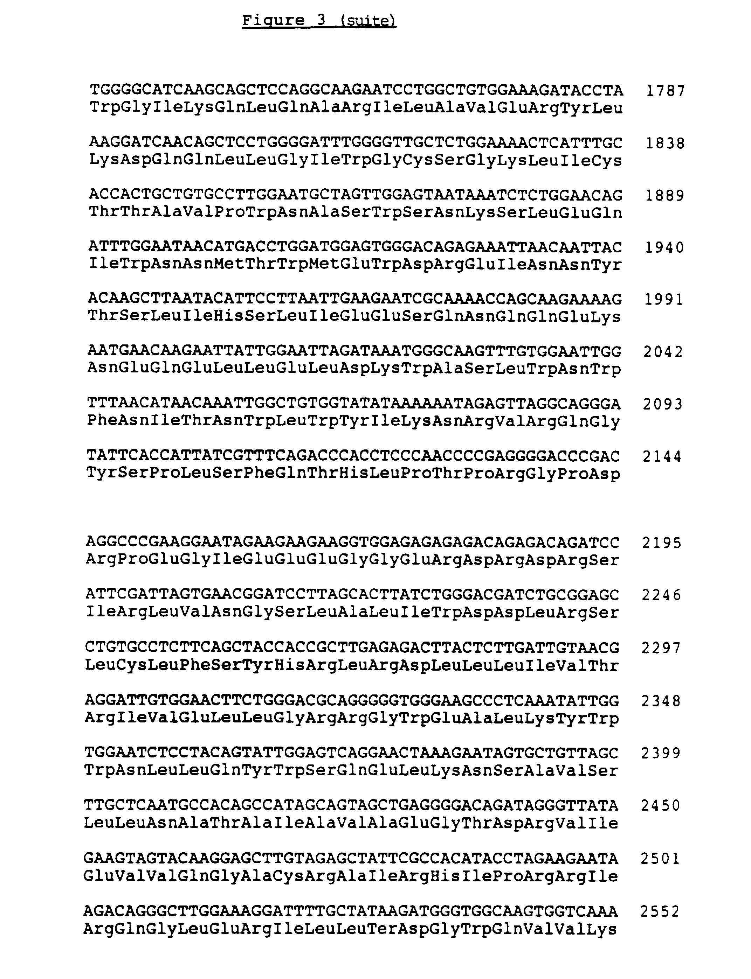

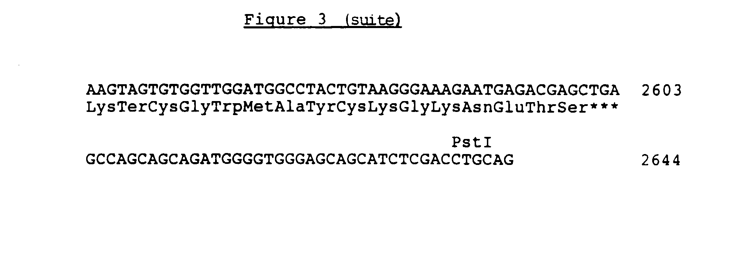

La Figure 3 présente la séquence nucléotidique du fragment *Pstl-Pstl* du vecteur pTGl163 qui comporte la séquence codant pour un précurseur d'un variant gp160-Bru non-clivable soluble (domaine transmembranaire absent) ainsi que la séquence en acides aminés de ce précurseur.

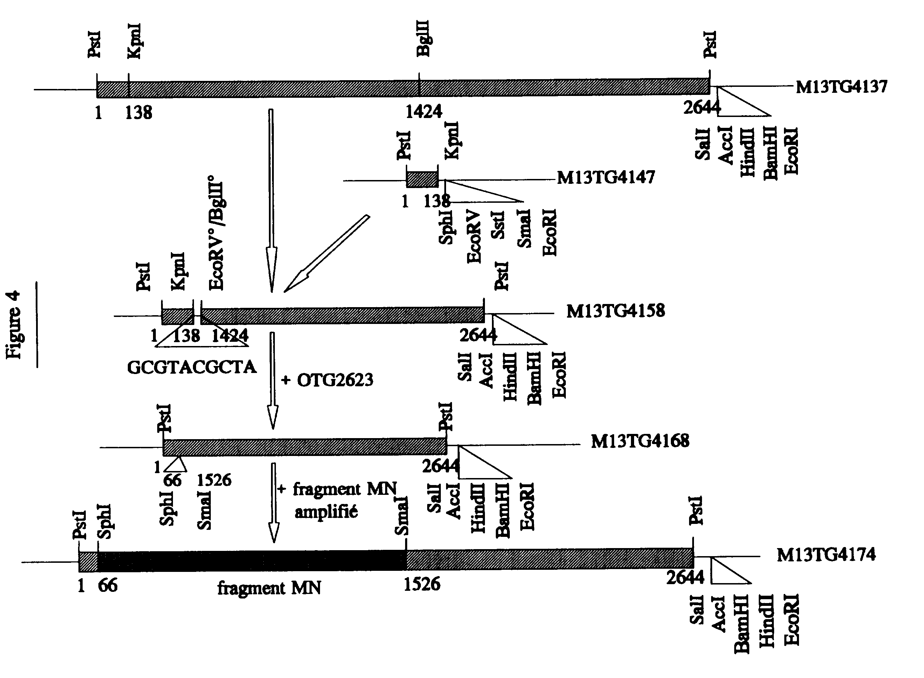

La Figure 4 schématise les étapes de la construction des bactériophages M13TG4168 et M13TG4174.

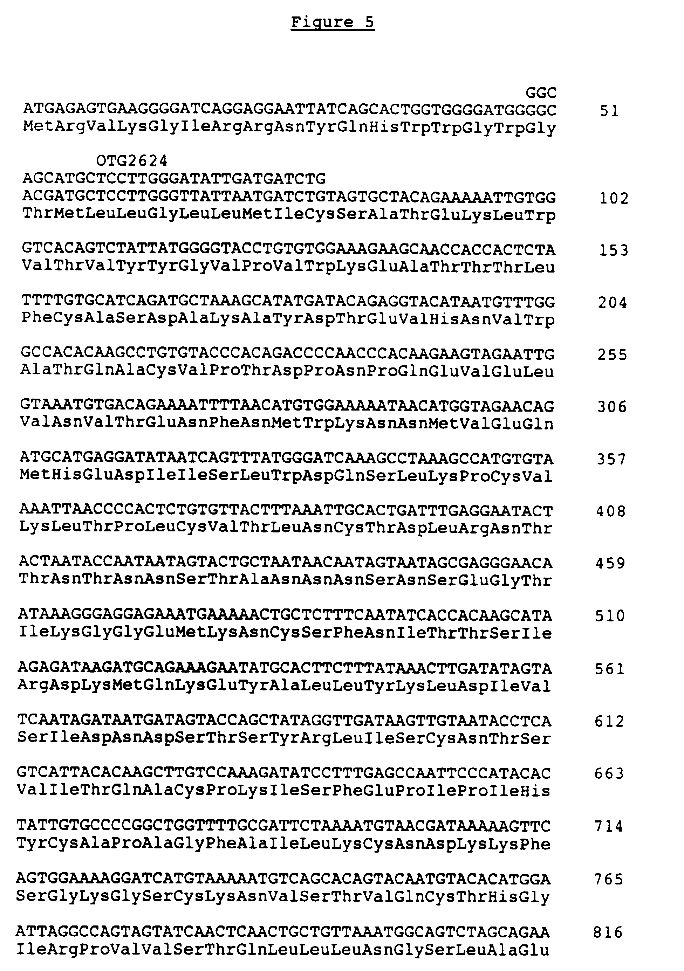

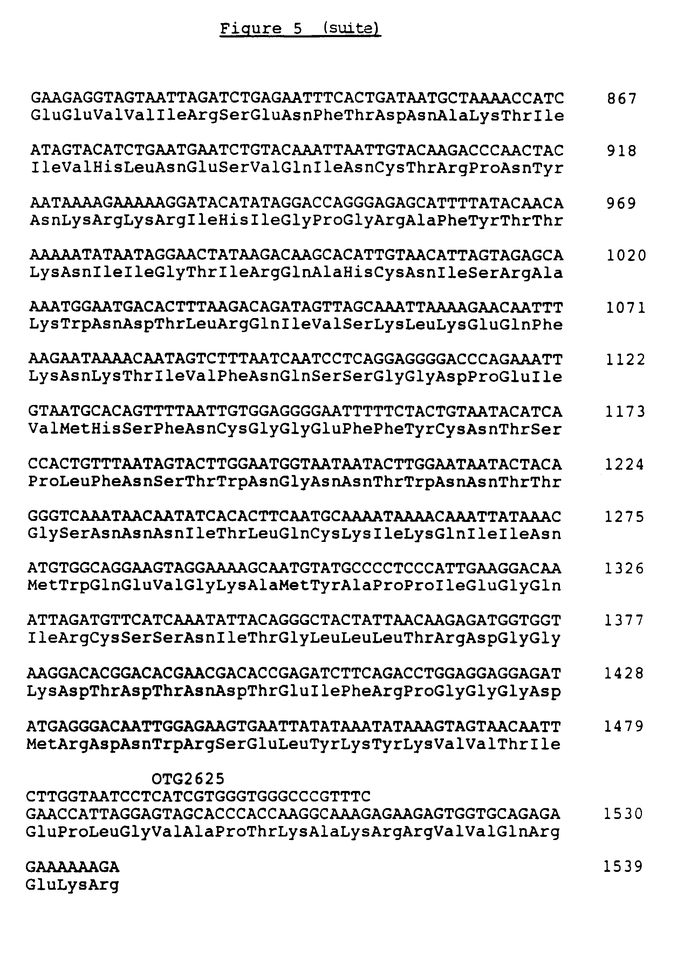

La Figure 5 présente la séquence nucléotidique codant pour le précurseur de la gp120-MN ainsi que la séquence en acides aminés de ce précurseur. Les oligonucléotides OTG2624 et OTG2625 destinés à l'amplification d'un fragment d'ADN codant au moins pour le troisième domaine hypervariable de la gp120-MN sont représentés au dessus de leur région d'hybridation.

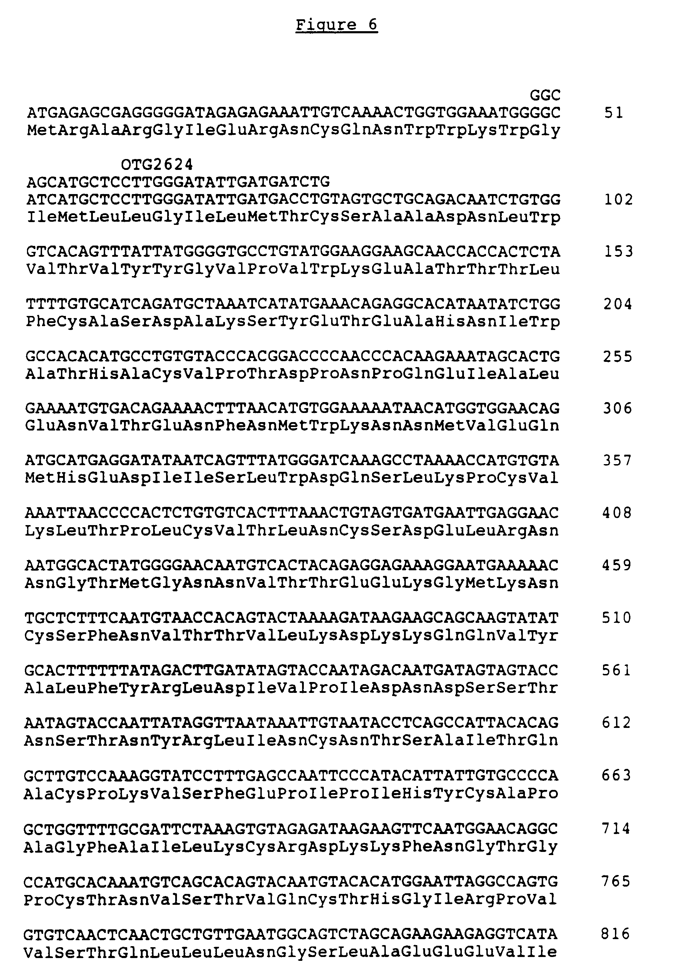

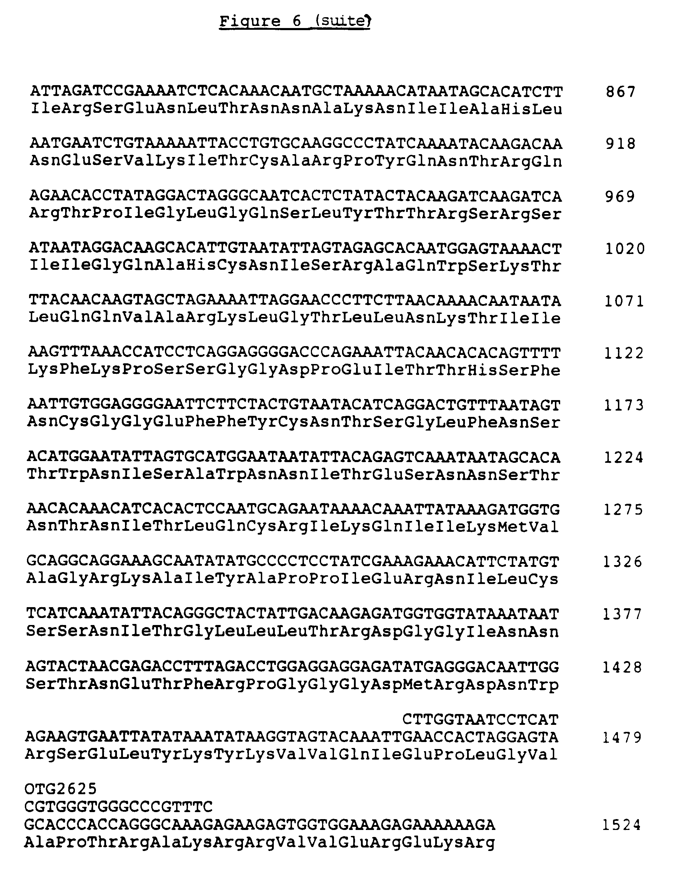

La Figure 6 présente la séquence nucléotidique codant pour le précurseur de la gp120-Eli ainsi que la séquence en acides aminés de ce précurseur. Les oligonucléotides OTG2624 et OTG2625 destinés à l'amplification d'un fragment d'ADN codant au moins pour le troisième domaine hypervariable de la gp120-MN sont représentés au dessus de leur région d'hybridation.

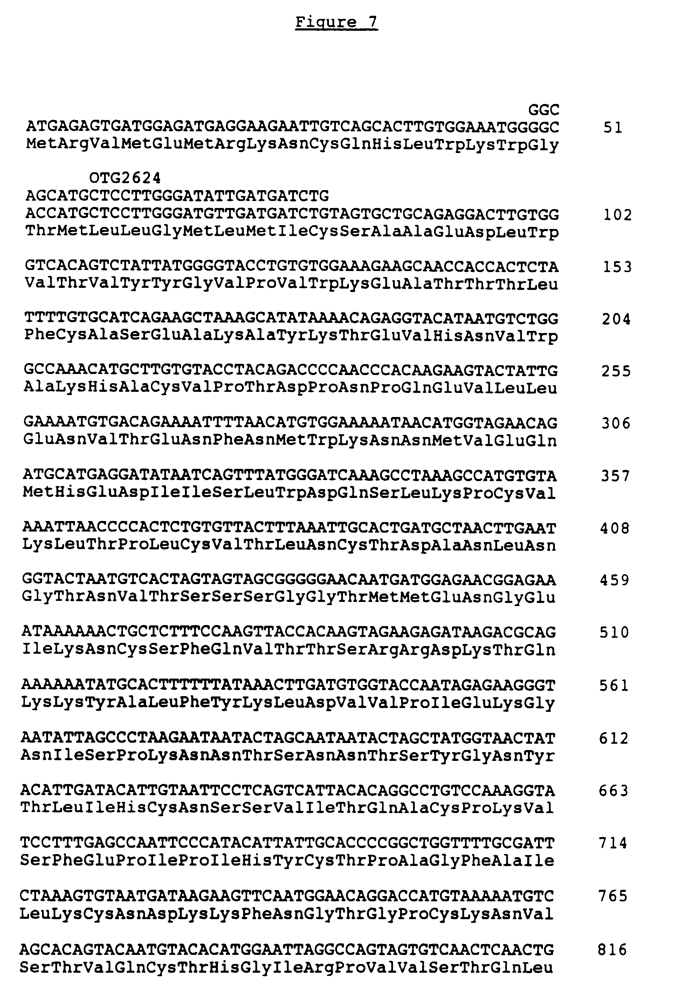

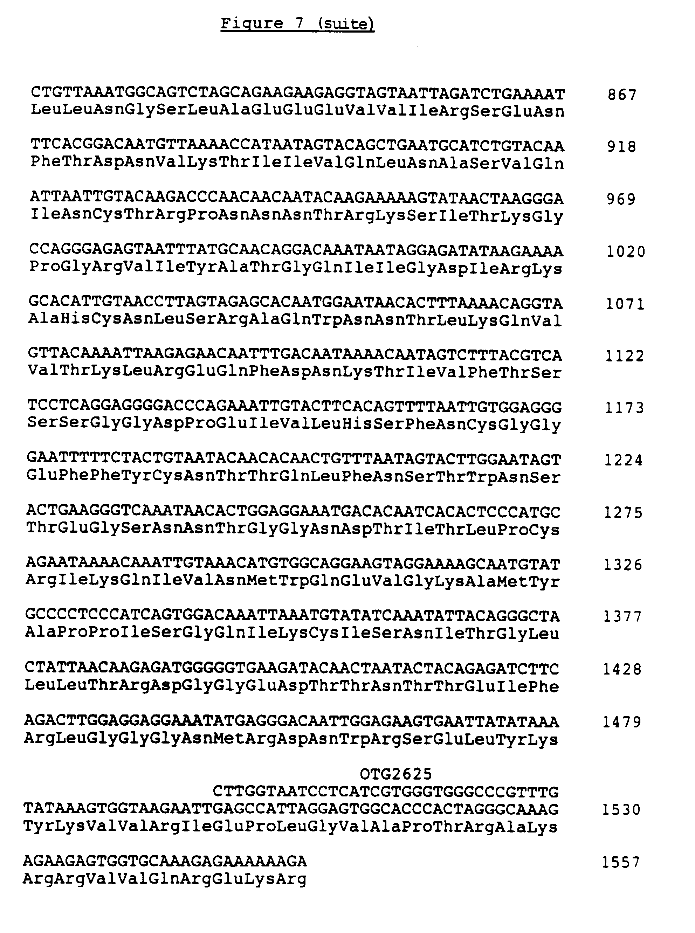

La Figure 7 présente la séquence nucléotidique codant pour le précurseur de la gp120-RF ainsi que la séquence en acides aminés de ce précurseur. Les oligonucléotides OTG2624 et OTG2625 destinés à l'amplification d'un fragment d'ADN codant au moins pour le troisième domaine hypervariable de la gp120-RF sont représentés au dessus de leur région d'hybridation.

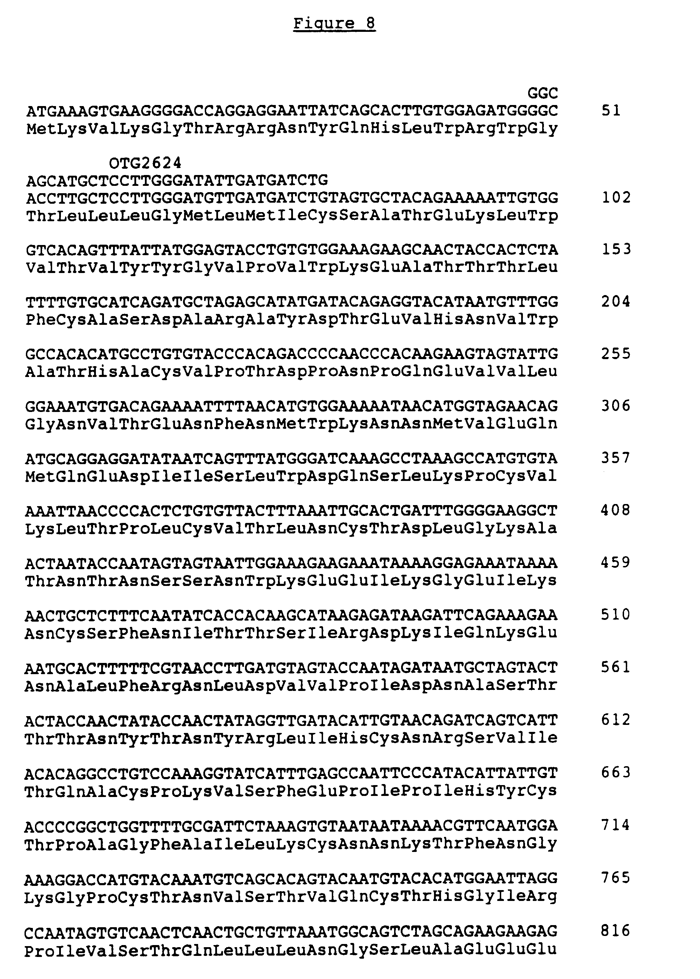

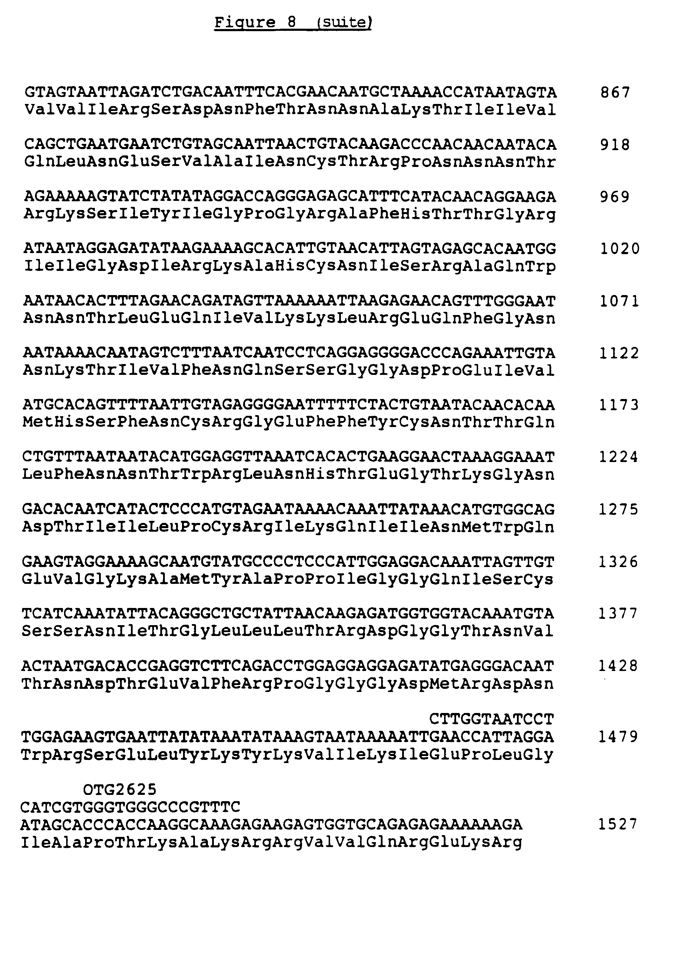

La Figure 8 présente la séquence nucléotidique codant pour le précurseur de la gp120-SF2C ainsi que la séquence en acides aminés de ce précurseur. Les oligonucléotides OTG2624 et OTG2625 destinés à l'amplification d'un fragment d'ADN codant au moins pour le troisième domaine hypervariable de la gp120-SF2C sont représentés au dessus de leur région d'hybridation.

Dans les exemples suivants, pour faciliter l'écriture et la compréhension, on entend par "séquence signal", une séquence signal incluant le résidu méthionine d'initiation de traduction.

### Exemple 1: Construction d'une cassette d'insertion porté par le bactériophage M13TG4168.

Tel que montré à la Figure 3, le fragment d'ADN *Pstl-Pstl* du plasmide pTG1163 décrit dans la demande de brevet EPA 245 136 comporte une séquence d'ADN codant pour un précurseur d'un variant gp160-Bru non-clivable et soluble. Ce fragment d'ADN *Pstl-Pstl* est inséré dans le bactériophage M13mp701 (décrit par M.P. Kieny et al. Gene (1983) 26 : 91) préalablement digéré par *Pstl* pour donner le bactériophage M13TG4137 (Figure 4). La numérotation des nucléotides du fragment *Pstl-Pstl* telle que indiquée à la Figure 3 sert de référence dans la suite de l'exemple 1.

Le plasmide pTG1163 est coupé par *Pstl* et *Kpnl* et le fragment d'ADN correspondant aux nucléotides 1 à 138 (Figure 3) est inséré dans le bactériophage M13TG130 (décrit par M.P. Kieny et al. (1983), supra) préalablement digéré par *Pstl* et *Kpnl.* On obtient ainsi le bactériophage M13TG4147 (Figure 4).

Le bactériophage M13TG4137 est coupé par *BglII,* traité par l'enzyme klenow (Boehringer Mannheim) pour obtenir des extrémités franches, puis coupé par *EcoRI.* Le fragment *EcoRI-BglII°* issu de cette digestion et comportant la séquence correspondant aux nucléotides 1424 à 2644 (Figure 3) est inséré dans le bactériophage M13TG4147 préalablement digéré par *EcoRV* et *EcoRI*. On obtient ainsi le bactériophage M13TG4158 qui comporte :
i) un fragment d'ADN correspondant aux nucléotides 1 à 138,
ii) la séquence restante du polylinker du bactériophage M13TG4147 c'est à dire ATCGCATGCG,
iii) un fragment d'ADN correspondant aux nucléotides 1424 à 2644.

Le bactériophage M13TG4158 simple brin anti-sens, sert de matrice pour une mutation-délétion effectuée en utilisant la trousse Amersham et l'oligonucléotide OTG2623 dont la séquence est la suivante :

SphI SmaI
GGGGGTGGAAATGGGGCA GCATGC AT CCCGGG CACAGAATCACGTGGTGC.

Cette mutagénèse permet simultanément de déléter un fragment de 184 paires de bases qui comporte les nucléotides 67 à 138, la séquence ATCGCATGCG et les nucléotides 1424 à 1525 et de créer les sites de coupure pour les enzymes *Sphl* en 5' et *Smal* en 3' qui sont insérés entre les nucléotides 66 et 1526. On obtient ainsi le bactériophage M13TG4168 qui comprend :
i) un fragment d'ADN correspondant aux nucléotides 1 à 66,
ii) la séquence d'ADN GCATGCATCCCG comportant les sites de clivage des enzymes *Sphl* et *Smal,* et
iii) un fragment d'ADN correspondant aux nucléotides 1526 à 2644.

### Exemple 2 : Construction d'une cassette d'expression destinée à la synthèse d'un variant gp160 hybride Bru-MN.

Tel que montré à la Figure 5, le fragment d'ADN codant pour la majeure partie d'un précurseur de la gpl20-MN est cloné par la technique d'amplification génique PCR (Polymérase Chain Reaction) mise en oeuvre à partir de l'ADN génomique de cellules humaines CEM infectées par la souche virale HIV1-MN. Ce clonage est effectué à l'aide des oligonucléotides OTG2624 et OTG2625 qui introduisent respectivement un site de coupure pour l'enzyme *Sphl* en 5' du fragment d'ADN amplifié et un site de coupure pour l'enzyme *Smal* en 3' du fragment d'ADN amplifié. Les séquences de ces oligonucléotides sont les suivantes :

SphI
OTG2624 : GGCA GCATGC TCCTTGGGATATTGATGATCTG

SmaI
OTG2625 : CTTTG CCCGGG TGGGTGCTACTCCTAATGGTTC

Le fragment d'ADN *Sphl-Smal* amplifié correspond aux nucléotides 53 à 1505 représentés à la Figure 5, compte tenu des modifications de séquence apportées par les oligonucléotides OTG2624 et OTG2625. Ce fragment est inséré dans le bactériophage M13TG4168 coupé par *Sphl* et *Smal.* On obtient ainsi le bactériophage M13TG4174 qui comporte un fragment d'ADN codant pour un précurseur d'une protéine gp160 hybride Bru-MN. Le fragment *Pstl* du bactériophage M13TG4174 qui code pour le variant gp160 hybride Bru-MN est inséré dans le plasmide de transfert pTG186poly (décrit par M.P. Kieny et al., Biotechnology, (1986) 4 : 790), en aval du promoteur E7.5k et à l'intérieur du gène du virus de la vaccine codant pour la thymidine kinase. On obtient ainsi le plasmide pTG5156.

Le plasmide pTG5156 est utilisé par la suite pour transférer le bloc d'expression de la protéine gp160 hybride Bru-MN dans le génome du virus de la vaccine, souche Copenhagen, selon la méthode décrite par M.P. Kieny et al. Nature (1984), 312, 163-166). On obtient ainsi le vecteur de la vaccine VVTG5156.

### Exemple 3 : Construction d'une cassette d'expression destinée à la synthèse d'un variant gp160 hybride Bru-Eli.

Le plasmide pTG186poly est coupé par *BamHI,* traité par l'enzyme Klenow, coupé par *Smal* pour déléter la majeure partie du polylinker, puis religué sur lui-même pour donner le plasmide pTG186PE. Le polylinker ne conserve plus que les sites de clivage pour les enzymes *Pstl, SalI* et *EcoRI.*

Le fragment d'ADN *Pstl-Pstl* du bactériophage M13TG4168 décrit, ci-dessus, qui comporte :
i) un fragment d'ADN correspondant aux nucléotides 1 à 66 du fragment d'ADN représenté sur la Figure 3,
ii) la séquence d'ADN GCATGCATCCCG comportant les sites de clivage des enzymes *Sphl* et *Smal,* et
iii) un fragment d'ADN correspondant aux nucléotides 1526 à 2644 du fragment d'ADN représenté à la Figure 3,
est inséré dans le plasmide pTG186PE prélablement coupé par *Pstl,* en aval du promoteur E7.5k et à l'intérieur du gène du virus de la vaccine codant pour la thymidine kinase. On obtient ainsi le plasmide pTG5160.

Tel que montré à la Figure 6, le fragment d'ADN codant pour la majeure partie d'un précurseur de la gp120-Eli est cloné par la technique d'amplification génique PCR mise en oeuvre à partir de l'ADN génomique de cellules humaines CEM infectées par du virus HIV1-Eli. Ce clonage est effectué à l'aide des oligonucléotides OTG2624 et OTG2625 qui introduisent respectivement un site coupure pour l'enzyme *Sphl* en 5' du fragment d'ADN amplifié et un site de coupure pour l'enzyme *Smal* en 3' du fragment d'ADN amplifié. Le fragment d'ADN *Sphl-Smal* amplifié correspond aux nucléotides 53 à 1490 représentés à la Figure 6, compte tenu des modifications de séquence apportées par les oligonucléotides OTG2624 et OTG2625. Ce fragment est inséré dans le bactériophage M13TG131 (décrit par M.P. Kieny et al. (1983), supra) préalablement coupé par *Sphl* et *Smal* pour donner le bactériophage M13TG4197. Puis le fragment *Sphl-Smal* du bactériophage M13TG4197 est inséré dans le plasmide de transfert pTG5160 (décrit ci-dessus) préalablement coupé par *Sphl* et *Smal.* On obtient ainsi le pTG5193.

Le plasmide pTG5193 est utilisé par la suite pour transférer le bloc d'expression de la protéine gp160 hybride Bru-Eli dans le génome du virus de la vaccine, souche Copenhagen, selon la méthode décrite par M.P. Kieny et al. (1984) supra. On obtient ainsi le vecteur de la vaccine VVTG5193.

### Exemple 4 : Construction d'une cassette d'expression destinée à la synthèse d'un variant gp160 hybride Bru-RF.

Tel que montré à la Figure 7, le fragment d'ADN codant pour la majeure partie d'un précurseur d'une protéine gp120-RF est cloné par la technique d'amplification génique PCR mise en oeuvre à partir de l'ADN génomique de cellules humaines CEM infectées par du virus HIV1-RF. Ce clonage est effectué à l'aide des oligonucléotides OTG2624 et OTG2625 qui introduisent respectivement un site de coupure pour l'enzyme *Sphl* en 5' du fragment d'ADN amplifié et un site de coupure pour l'enzyme *Smal* en 3' du fragment d'ADN amplifié. Le fragment d'ADN *Sphl-Smal* amplifié correspond aux nucléotides 53 à 1523 représentés à la Figure 7, compte tenu des modifications de séquence apportées par les oligonucléotides OTG2624 et OTG2625. Ce fragment est inséré dans le bactériophage M13TG131 préalablement coupé par *Sphl* et *Smal* pour donner le bactériophage M13TG4198. Puis le fragment *Sphl-Smal* du bactériophage M13TG4198 est inséré dans le plasmide de transfert pTG5160 (décrit à l'exemple 3) préalablement coupé par *Sphl* et *Smal*. On obtient ainsi le pTG5194.

Le plasmide pTG5194 est utilisé par la suite pour transférer le bloc d'expression de la protéine gp160 hybride Bru-RF dans le génome du virus de la vaccine, souche Copenhagen, selon la méthode décrite par M.P. Kieny et al. (1984) supra. On obtient ainsi le vecteur de la vaccine VVTG5194.

### Exemple 5 : Construction d'une cassette d'expression destinée à la synthèse d'un variant gp160 hybride Bru-SF2C.

Tel que montré à la Figure 8, le fragment d'ADN codant pour la majeure partie d'un précurseur de la gp120-SF2C est cloné par la technique d'amplification génique PCR mise en oeuvre à partir de l'ADN génomique de cellules humaines CEM infectées par du virus HIV1-SF2C. Ce clonage est effectué à l'aide des oligonucléotides OTG2624 et OTG2625 qui introduisent respectivement un site de coupure pour l'enzyme *Sphl* en 5' du fragment d'ADN amplifié et un site de coupure pour l'enzyme *Smal* en 3' du fragment d'ADN amplifié. Le fragment d'ADN *Sphl-Smal* amplifié correspond aux nucléotides 53 à 1493 représentés à la Figure 8, compte tenu des modifications de séquence apportées par les oligonucléotides OTG2624 et OTG2625. Puis ce fragment est inséré dans le bactériophage M13TG131 préalablement coupé par *Sphl* et *Smal* pour donner le bactériophage M13TG4199. Le fragment *Sphl-Smal* du bactériophage M13TG4199 est inséré dans le plasmide de transfert pTG5160 (décrit à l'exemple 3) préalablement coupé par *Sphl* et *Smal.* On obtient ainsi le pTG5195.

Le plasmide pTG5195 est utilisé par la suite pour transférer le bloc d'expression de la protéine gp160 hybride Bru-SF2C dans le génome du virus de la vaccine, souche Copenhagen, selon la méthode décrite par M.P. Kieny et al. (1984) supra. On obtient ainsi le vecteur de la vaccine VVTG5195.

### Exemples 6 à 9 : Production et purification des gp160 hybrides Bru-MN, Bru-Eli, Bru-RF et Bru SF2C :

Des cellules BHK-21 sont cultivées dans un milieu GMEM (Gibco) supplémenté avec 10% de sérum de veau foetal (SVF). Lorsque les cellules sont à confluence (5,8x10⁶cellules/ml), le milieu de culture est enlevé et le tapis cellulaire est lavé 2 fois avec 50 ml de PBS (Dubelcco's phosphate buffer salt Seromed). Puis du milieu GMEM frais sans SVF est rajouté. On ajoute alors un des virus de la vaccine VVTG5156, VVTG5193, VVTG5194 et VVTG5195 décrits ci-dessus dans les exemples 2 à 5, à une infectivité de 1 ufp/cellule (unité formant plages), et l'infection est poursuivie pendant 72 h. Le lysat est alors centrifugé 20 min. à 10 000 g pour éliminer les débris cellulaires et on récupère le surnageant contenant entre autre une gp160 hybride et des virions.

A 20 ml de surnageant de culture obtenu comme précédemment décrit, on ajoute 1.3 ml d'une solution de chlorure de zinc (ZnCl₂) à 1 M pour avoir une concentration finale en ZnCl₂ de 60 mM. Le mélange est laissé 1 h dans de la glace ; puis le surnageant de précipitation est récupéré après centrifugation à 3000 t/min. pendant 20 min. dans une centrifugeuse Minifuge RF Heraeus. Cette méthode élimine par précipitation les virions ainsi que la majorité des protéines contaminantes. Dans chaque cas, on obtient ainsi une solution purifiée du variant gp160 hybride.

## Revendications

1. Une méthode pour construire une cassette d'expression comportant une unité d'ADN codant soit pour un précurseur d'un variant gp160 hybride, non-clivable et soluble, soit pour un variant gp160 hybride, non-clivable et soluble ; ledit variant gp160 ayant une séquence en acides aminés comprenant :
i) une première région dérivée de la gp160 d'une première souche du virus HIV et située dans cette dernière gp160 de l'acide aminé en position X à l'acide aminé en position Y, X étant un nombre de 1 à 271 et Y étant un nombre de 306 à 482 ;
ii) une deuxième région dérivée d'un variant gp160 soluble, non-clivable de type A ayant pour origine une deuxième souche du virus HIV et située dans ce dernier variant gp160 de l'acide aminé en position Y + 1 à l'acide aminé en position C-terminale, Y étant tel que défini ci-dessus ; et
iii) quand X est différent de 1, une troisième région dérivée de la gp160 de ladite deuxième souche du virus HIV et située dans cette dernière gp160 de l'acide aminé en position 1 à l'acide aminé en position X - 1, X étant tel que défini ci-dessus ;
ladite méthode comprenant :
a) l'acte de cloner un fragment d'ADN codant pour ladite première région ; et
b) l'acte d'insérer le fragment d'ADN cloné en a) dans un site d'une cassette qui comprend ;
m) en amont du site et en séquence :
i) un promoteur,
ii) un codon d'initiation de traduction,
iii) en option, une première région d'ADN codant pour un peptide signal, et
iv) quand X est tel que défini ci-dessus mais différent de 1, une deuxième région d'ADN codant pour ladite troisième région de la séquence en acides aminés dudit variant gp 160 hybride ; et
n) en aval du site en séquence :
i) quand Y est tel que défini ci-dessus, une troisième région d'ADN codant pour ladite deuxième région de la séquence en acides aminés dudit variant gp160 hybride et
ii) un codon de terminaison de traduction ;
pour obtenir une cassette d'expression comportant ladite unité d'ADN.

2. Une méthode selon la revendication 1, caractérisée en ce que l'unité d'ADN code pour un précurseur d'un variant gp160 hybride, non clivable et soluble.

3. Une méthode selon la revendication 1, caractérisée en ce que la deuxième région dérivée d'un variant de gp160 soluble, non clivable est de type A'.

4. Une méthode selon la revendication 1 ou 3, caractérisée en ce que la deuxième souche du virus HIV est la souche HIV 1-Bru.

5. Une méthode selon l'une des revendications 1, 3 ou 4, caractérisée en ce que la première souche du virus HIV est sélectionnée parmi les souches HIV-1 MN, HIV-1 Eli, HIV-1 RF, HIV-1 SF2C et HIV-1 SC.

6. Une méthode selon la revendication 5, caractérisée en ce que la première souche du virus HIV est la souche HIV-MN.

7. Une méthode selon l'une des revendications 1 et 3 à 6, caractérisée en ce que Y est un nombre de 450 à 482 et la deuxième région dérivée d'un variant de gp160 soluble non clivable, est de type A'.

8. Une méthode selon l'une des revendications 1 et 3 à 7, caractérisée en ce que X est un nombre de 1 à 97 et la deuxième région dérivée d'un variant de gp160 soluble non clivable, est de type A'.

9. Une méthode selon l'une des revendications 1 et 3 à 8, caractérisée en ce que X est un nombre de 1 à 97 et la deuxième région dérivée d'un variant de gp160 soluble non clivable, est de type A', et on clone un fragment d'ADN codant pour ladite première région par la technique PCR à partir de l'ADN génomique de cellules infectées par ladite première souche du virus HIV.

10. Un variant gp160 hybride, non clivable et soluble, ayant une séquence en acides aminés qui comprend :
i) une première région de la gp160 d'une première souche du virus HIV et située dans cette dernière gp160 de l'acide aminé en position X à l'acide aminé en position Y, X étant un nombre de 1 à 271 et Y étant un nombre de 306 à 482 ;
ii) une deuxième région dérivée d'un variant gp160 soluble, non clivable de type A ayant pour origine une deuxième souche du virus HIV et située dans ce dernier variant gp160 de l'acide aminé en position Y + 1 à l'acide aminé en position C-terminale, Y étant tel que défini ci-dessus et
iii) quand X est différent de 1, une troisième région dérivée de la gp160 de ladite deuxième souche du virus HIV et située dans cette dernière gp160 de l'acide aminé en position 1 à l'acide aminé en position X - 1, X étant tel que défini ci-dessus.

11. Un variant gp160 hybride selon la revendication 10, caractérisé en ce que la deuxième région dérivée d'un variant de gp160 soluble non clivable est de type A'.

12. Un variant gp160 hybride selon l'une des revendications 10 ou 11, caractérisé en ce que la deuxième souche du virus HIV est la souche HIV 1-Bru.

13. Un variant gp160 hybride selon l'une des revendications 10 à 12, caractérisé en ce que la première souche du virus HIV est sélectionnée parmi les souches HIV-1 MN, HIV-1 Eli, HIV-1 RF, HIV-1 SF2C et HIV-1 SC.

14. Un variant gp160 hybride selon la revendication 13, caractérisé en ce que la première souche du virus HIV est la souche HIV-1 MN.

15. Un variant gp160 hybride selon l'une des revendications 10 à 14, caractérisé en ce que Y est un nombre de 450 à 482 et la deuxième région dérivée d'un variant de gp160 soluble non clivable, est de type A'.

16. Un variant gp160 hybride selon l'une des revendications 10 à 15, caractérisé en ce que X est un nombre de 1 à 97 et la deuxième région dérivée d'un variant de gp160 soluble non clivable, est de type A'.

17. Une cassette d'expression obtenue par une méthode selon n'importe laquelle des revendications 1 à 9.

18. Une cassette d'expression comportant une unité d'ADN codant soit pour un précurseur d'un variant gp160, soit pour un variant gp160, tel que défini dans l'une des revendications 10 à 16, et les éléments nécessaires à l'expression de ladite unité d'ADN.

19. Une cellule transformée par une cassette d'expression selon la revendication 17 ou 18.

20. Un vecteur viral dans le génome duquel est inséré une cassette d'expression selon la revendication 17 ou 18.

21. Un procédé pour produire un variant gp160 selon n'importe laquelle des revendications 10 à 16, qui comprend l'acte de cultiver une cellule selon la revendication 19 et l'acte de récolter ledit variant à partir de la culture.

22. Un procédé pour produire un variant gp160 selon n'importe laquelle des revendications 10 à 16, qui comprend l'acte d'infecter une culture de cellules avec un vecteur viral selon la revendication 20 et l'acte de récolter ledit variant à partir de la culture.

23. Une composition pharmaceutique destinée à la prévention ou au traitement du Sida, qui comprend, à titre d'agent thérapeutique, un variant gp160 selon n'importe laquelle des revendications 10 à 16.

24. Une composition pharmaceutique destinée à la prévention ou au traitement du Sida, qui comprend, à titre d'agent thérapeutique, un vecteur viral selon la revendication 20.

## Patentansprüche

1. Verfahren zur Herstellung einer Expressions-Cassette, die eine DNA-Einheit umfaßt, die entweder für einen Vorläufer einer nicht-schneidbaren und löslichen Hybrid-gp160-Variante oder für eine nicht-schneidbare und lösliche Hybrid-gp160-Variante codiert, wobei die genannte gp160-Variante eine Aminosäure-Sequenz aufweist, die umfaßt:
i) eine erste Region, die von der gp160 eines ersten Stammes des HIV-Virus abgeleitet ist und in der zuletzt genannten gp160 von der Aminosäure in der Position X bis zur Aminosäure in der Position Y angeordnet ist, wobei X eine Zahl von 1 bis 271 und Y eine Zahl von 306 bis 482 bedeuten;
ii) eine zweite Region, die von einer nicht-schneidbaren löslichen gp160-Variante vom Typ A abgeleitet ist, die aus einem zweiten Stamm des HIV-Virus stammt und in dieser letztgenannten gp160-Varianter von der Aminosäure in der Position Y + 1 bis zur Aminosäure in der C-terminalen Position angeordnet ist, wobei Y wie oben definiert ist; und
iii) eine dritte Region, wenn X von 1 verschieden ist, die von der gp160 des genannten zweiten Stammes des HIV-Virus abgeleitet ist und in der zuletzt genannten gp160 von der Aminosäure in der Position 1 bis zur Aminosäure in der Position X - 1 angeordnet ist, wobei X wie oben definiert ist;
wobei das genannte Verfahren umfaßt:
a) die Klonierung eines DNA-Fragments, das für die genannte erste Region codiert; und
b) die Insertion des in der Stufe (a) klonierten DNA-Fragments in eine Stelle einer Cassette, die umfaßt:
m) stromaufwärts von der Stelle und aufeinanderfolgend:
i) einen Promotor,
ii) ein Translations-lnitiierungscodon,
iii) gegebenenfalls eine erste DNA-Region, die für ein Signal-Peptid codiert, und
iv) eine zweite DNA-Region, wenn X wie oben definiert ist, die für die genannte dritte Region der Aminosäure-Sequenz der genannten Hybrid-gp160-Variante codiert; und
n) stromabwärts von der Stelle und aufeinanderfolgend:
i) eine dritte DNA-Region, wenn Y wie oben definiert ist, die für die genannte zweite Region der Aminosäure-Sequenz der genannten Hybrid-gp160-Variante codiert, und
ii) ein Translations-Terminationscodon;
unter Bildung einer Expressions-Cassette, welche die genannte DNA-Einheit umfaßt.

2. Verfahren nach Anspruch 1, dadurch gekennzeichnet, daß die DNA-Einheit für einen Vorläufer einer nicht-schneidbaren und löslichen Hybrid-gp160-Variante codiert.

3. Verfahren nach Anspruch 1, dadurch gekennzeichnet, daß die zweite Region, die von einer nicht-schneidbaren und löslichen gp160-Variante abgeleitet ist, eine solche vom Typ A' ist.

4. Verfahren nach Anspruch 1 oder 3, dadurch gekennzeichnet, daß der zweite Stamm des HIV-Virus der Stamm HIV 1-Bru ist.

5. Verfahren nach einem der Ansprüche 1, 3 oder 4, dadurch gekennzeichnet, daß der erste Stamm des HIV-Virus unter den Stämmen HIV-1 MN, HIV-1 Eli, HIV-1 RF, HIV-1 SF2C und HIV-1 SC ausgewählt wird.

6. Verfahren nach Anspruch 5, dadurch gekennzeichnet, daß der erste Stamm des HIV-Virus der Stamm HIV-1 MN ist.

7. Verfahren nach einem der Ansprüche 1 und 3 bis 6, dadurch gekennzeichnet, daß Y eine Zahl von 450 bis 482 ist und die zweite Region, die von einer nicht-schneidbaren und löslichen gp160-Variante abgeleitet ist, eine solche vom Typ A' ist.

8. Verfahren nach einem der Ansprüche 1 und 3 bis 7, dadurch gekennzeichnet, daß X eine Zahl von 1 bis 97 ist und die zweite Region, die von einer nicht-schneidbaren und löslichen gp160-Variante abgeleitet ist, eine solche vom Typ A' ist.

9. Verfahren nach einem der Ansprüche 1 und 3 bis 8, dadurch gekennzeichnet, daß X eine Zahl von 1 bis 97 ist und die zweite Region, die von einer nicht-schneidbaren und löslichen gp160-Variante abgeleitet ist, eine solche vom Typ A' ist und daß man ein DNA-Fragment, das für die genannte erste Region codiert, kloniert nach der PCR-Methode, wobei man von der Genom-DNA von Zellen ausgeht, die durch den genannten ersten Stamm des HIV-Virus infiziert sind.

10. Nicht-schneidbare und lösliche Hybrid-gp160-Variante, die eine Sequenz von Aminosäuren aufweist, die umfaßt:
i) eine erste Region der gp160 eines ersten Stammes des HIV-Virus, die in der zuletzt genannten gp160 von der Aminosäure in der Position X bis zur Aminosäure in der Position Y angeordnet ist, wobei X eine Zahl von 1 bis 271 und Y eine Zahl von 306 bis 482 darstellen;
ii) eine zweite Region, die von einer nicht-schneidbaren und löslichen gp160-Variante vom Typ A abgeleitet ist, der aus einem zweiten Stamm des HIV-Virus stammt und in der zuletzt genannten gp160-Variante von der Aminosäure in der Position Y + 1 bis zur Aminosäure in der C-terminalen Position angeordnet ist, wobei Y wie oben definiert ist, und
iii) eine dritte Region, wenn X von 1 verschieden ist, die von der gp160 des genannten zweiten Stammes des HIV-Virus abgeleitet ist und in der zuletzt genannten gp160 von der Aminosäure in der Position 1 bis zur Aminosäure in der Position X - 1 angeordnet ist, wobei X wie oben definiert ist.

11. Hybrid-gp160-Variante nach Anspruch 10, dadurch gekennzeichnet, daß die zweite Region, die von einer nicht-schneidbaren und löslichen gp160-Variante abgeleitet ist, eine solche vom Typ A' ist.

12. Hybrid-gp160-Variante nach einem der Ansprüche 10 oder 11, dadurch gekennzeichnet, daß der zweite Stamm des HIV-Virus der Stamm HIV 1-Bru ist.

13. Hybrid-gp160-Variante nach einem der Ansprüche 10 bis 12, dadurch gekennzeichnet, daß der erste Stamm des HIV-Virus unter den Stämmen HIV-1 MN, HIV-1 Eli, HIV-1 RF, HIV-1 SF2C und HIV-1 SC ausgewählt wird.

14. Hybrid-gp160-Variante nach Anspruch 13, dadurch gekennzeichnet, daß der erste Stamm des HIV-Virus der Stamm HIV-1 MN ist.

15. Hybrid-gpl 60-Variante nach einem der Ansprüche 10 bis 14, dadurch gekennzeichnet, daß Y eine Zahl von 450 bis 482 ist und die zweite Region, die von einer nicht-schneidbaren und löslichen gp160-Variante abgeleitet ist, eine solche vom Typ A' ist.

16. Hybrid-gp160-Variante nach einem der Ansprüche 10 bis 15, dadurch gekennzeichnet, daß X eine Zahl von 1 bis 97 ist und die zweite Region, die von einer nicht-schneidbaren und löslichen gp160-Variante abgeleitet ist, eine solche vom Typ A' ist.

17. Expressions-Cassette, wie sie nach einem Verfahren nach einem der Ansprüche 1 bis 9 erhalten wird.

18. Expressions-Cassette, die umfaßt eine DNA-Einheit, die entweder für einen Vorläufer einer gp160-Variante oder für eine gp160-Variante, wie in einem der Ansprüche 10 bis 16 defineirt, codiert, und die für die Expression der genannten DNA-Einheit erforderlichen Elemente.

19. Zelle, die durch eine Expressions-Cassette nach Anspruch 17 oder 18 transformiert worden ist.

20. Viraler Vektor, in dessen Genom eine Expressions-Cassette nach Anspruch 17 oder 18 inseriert ist.

21. Verfahren zur Herstellung einer gp160-Variante nach irgendeinem der Ansprüche 10 bis 16, das umfaßt die Kultivierung einer Zelle nach Anspruch 19 und die Gewinnung (Abtrennung) der genannten Variante aus der Kultur.

22. Verfahren zur Herstellung einer gp160-Variante nach irgendeinem der Ansprüche 10 bis 16, das umfaßt das Infizieren einer Zellen-Kultur mit einem viralen Vektor nach Anspruch 20 und die Gewinnung (Abtrennung) der genannten Variante aus der Kultur.

23. Pharmazeutische Zusammensetzung zur Verhinderung oder Behandlung von Aids, die als therapeutisches Agens (Wirkstoff) eine gp160-Variante nach einem der Ansprüche 10 bis 16 enthält.

24. Pharmazeutische Zusammensetzung zur Verhinderung oder Behandlung von Aids, die als therapeutisches Agens (Wirkstoff) einen viralen Vektor nach Anspruch 20 enthält.

## Claims

1. A method for constructing an expression cassette containing a DNA unit coding either for a precursor of a non-cleavable and soluble hybrid gp160 variant, or for a non-cleavable and soluble hybrid gp160 variant; the said' gp160 variant having an amino acid sequence comprising:
i) a first region derived from the gp160 of a first strain of HIV virus and located in this latter gp160 from the amino acid at position X to the amino acid at position Y, X being a number from 1 to 271 and Y being a number from 306 to 482;
ii) a second region derived from a type A non-cleavable, soluble gp160 variant originating from a second strain of HIV virus and located in this latter gp160 variant from the amino acid at position Y+1 to the amino acid at the C-terminal position, Y being as defined above; and
iii) when X is other than 1, a third region derived from the gp160 of the said second strain of HIV virus and located in this latter gp160 from the amino acid at position 1 to the amino acid at position X-1, X being as defined above;
the said method comprising:
a) the act of cloning a DNA fragment coding for the said first region; and
b) the act of inserting the DNA fragment cloned in a) into a site of a cassette which comprises:
m) upstream of the site and in sequence:
i) a promotor,
ii) a translation initiation codon,
iii) optionally, a first DNA region coding for a signal peptide, and
iv) when X is as defined above but other than 1, a second DNA region coding for the said third region of the amino acid sequence of the said hybrid gp160 variant; and
n) downstream of the site, in sequence:
i) when Y is as defined above, a third DNA region coding for the said second region of the amino acid sequence of the said hybrid gp160. variant, and
ii) a translation termination codon;
to obtain an expression cassette containing the said DNA unit.

2. A method according to Claim 1, characterized in that the DNA unit codes for a precursor of a non-cleavable and soluble hybrid gp160 variant.

3. A method according to Claim 1, characterized in that the second region derived from a non-cleavable, soluble gp160 variant is of type A'.

4. A method according to Claim 1 or 3, characterized in that the second strain of HIV virus is strain HIV-1 Bru.

5. A method according to one of Claims 1, 3 and 4, characterized in that the first strain of HIV virus is selected from the strains HIV-1 MN, HIV-1 Eli, HIV-1 RF, HIV-1 SF2C and HIV-1 SC.

6. A method according to Claim 5, characterized in that the first strain of HIV virus is the strain HIV-1 MN.

7. A method according to one of Claims 1 and 3 to 6, characterized in that Y is a number from 450 to 482 and the second region, derived from a non-cleavable, soluble gp160 variant, is of type A'.

8. A method according to one of Claims 1 and 3 to 7, characterized in that X is a number from 1 to 97 and the second region, derived from a non-cleavable, soluble gp160 variant, is of type A'.

9. A method according to one of Claims 1 and 3 to 8, characterized in that X is a number from 1 to 97 and the second region, derived from a non-cleavable, soluble gp160 variant, is of type A', and a DNA fragment coding for the said first region is cloned by the PCR technique from the genomic DNA of cells infected with the said first strain of HIV virus.

10. A non-cleavable and soluble hybrid gp160 variant having an amino acid sequence which comprises:
i) a first region of the gp160 of a first strain of HIV virus and located in this latter gp160 from the amino acid at position X to the amino acid at position Y, X being a number from 1 to 271 and Y being a number from 306 to 482;
ii) a second region derived from a type A non-cleavable, soluble gp160 variant originating from a second strain of HIV virus and located in this latter gp160 variant from the amino acid at position Y+1 to the amino acid at the C-terminal position, Y being as defined above; and
iii) when X is other than 1, a third region derived from the gp160 of the said second strain of HIV virus and located in this latter gp160 from the amino acid at position 1 to the amino acid at position X-1, X being as defined above.

11. A hybrid gp160 variant according to Claim 10, characterized in that the second region, derived from a non-cleavable, soluble gp160 variant, is of type A'.

12. A hybrid gp160 variant according to one of Claims 10 and 11, characterized in that the second strain of HIV virus is the strain HIV-1 Bru.

13. A hybrid gp160 variant according to one of Claims 10 to 12, characterized in that the first strain of HIV virus is selected from the strains HIV-1 MN, HIV-1 Eli, HIV-1 RF, HIV-1 SF2C and HIV-1 SC.

14. A hybrid gp160 variant according to Claim 13, characterized in that the first strain of HIV virus is the strain HIV-1 MN.

15. A hybrid gp160 variant according to one of Claims 10 to 14, characterized in that Y is a number from 450 to 482 and the second region, derived from a non-cleavable, soluble gp160 variant, is of type A'.

16. A hybrid gp160 variant according to one of Claims 10 to 15, characterized in that X is a number from 1 to 97 and the second region, derived from a non-cleavable, soluble gp160 variant, is of type A'.

17. An expression cassette obtained by a method according to any one of Claims 1 to 9.

18. An expression cassette containing a DNA unit-coding either for a precursor of a gp160 variant, or for a gp160 variant as defined in one of Claims 10 to 16, and the elements needed for expression of the said DNA unit.

19. A cell transformed with an expression cassette according to Claim 17 or 18.

20. A viral vector, into the genome of which an expression cassette according to Claim 17 or 18 is inserted.

21. A process for producing a gp160 variant according to any one of Claims 10 to 16, which comprises the act of culturing a cell according to Claim 19 and the act of harvesting the said variant from the culture.

22. A process for producing a gp160 variant according to any one of Claims 10 to 16, which comprises the act of infecting a cell culture with a viral vector according to Claim 20 and the act of harvesting the said variant from the culture.

23. A pharmaceutical composition intended for the prevention or treatment of AIDS, which comprises as therapeutic agent a gp160 variant according to any one of Claims 10 to 16.

24. A pharmaceutical composition intended for the prevention or treatment of AIDS, which comprises as therapeutic agent a viral vector according to Claim 20.
